# EUROPEAN PATENT SPECIFICATION

(11) **EP 3 241 509 B1**
(45) Date of publication and mention of the grant of the patent: **10.08.2022**
(21) Application number: 16168081.4
(22) Date of filing: 03.05.2016
(51) Int. Cl.: A61B 17/28

(54) **INSTRUMENT FOR COSMETIC SURGERY**
INSTRUMENT FÜR DIE SCHÖNHEITSCHIRURGIE
INSTRUMENT DE CHIRURGIE COSMÉTIQUE

(43) Date of publication of application: 08.11.2017
(73) Proprietor: Gualdi, Alessandro, 20121 Milano (IT)
(72) Inventor: Gualdi, Alessandro, 20121 Milano (IT)
(74) Representative: Colombo, Stefano Paolo

(56) References cited:
- EP-A1- 0 792 621
- WO-A2-01/21231
- DE-A1- 19 924 974
- DE-C- 527 109
- US-A1- 2013 090 681

## Description

The present invention relates to a surgical instrument, in particular an instrument for cosmetic surgery.

In the wake of an increasing number of requests on the part of patients, cosmetic surgeons are continually searching for face-lifting techniques which are less invasive, safer and involve a shorter recovery time after an operation.

In present-day society many persons wish to appear younger and to combat the effects of ageing, without losing their natural appearance. However, most patients wanting to undergo facial cosmetic surgery operations are willing to accept only short interruptions in their own social and working activities. Consequently, in the last few years various surgical procedures which are minimally invasive have been developed and various non-surgical techniques have also been proposed, with the aim of providing a visible, but natural change, with minimum risks, reducing at the same time the duration of the interruption in the social and working activities of the patient.

Even though various new techniques for facial rejuvenation have been proposed and adopted, the inventor considers that face-lifting still represents one of the best solutions.

The subperiosteal procedures produce good results in the long term, but on occasions patients complain about swelling which may last up to six months.

According to the present disclosure a technique has been devised where a minor incision is made within the hair line in the temple region. A blunt dissection is performed through this minor incision using a special surgical instrument in the caudal direction towards the outer canthus in a cleavage plane between the superficial and deep temple fascia. Proceeding in the caudal direction the subfascial pre-zygomatic space (sub-SMAS) is acted on using the instrument according to the invention and, using blunt dissection, lysis of the zygomatic-malar ligament is performed. At this point, still using the aforementioned instrument according to the present invention, the overlying tissue is engaged (SMAS, orbicularis muscle and malar bag) in a non-invasive manner with a straight needle suture, by means of a transcutaneous manoeuvre, allowing repositioning of the flap in the cranial position. The suture loop is taken up again by the instrument according to the invention and is extracted in the region of the initial incision and fastened deep at the aponeurosis of the temporal muscle.

In order to employ the aforementioned technique a surgical instrument comprising two arms pivoted to form substantially a first-class lever and a guide channel for guiding a straight surgical needle, towards the distal end of the instrument, is provided. Each arm comprises a plate at the distal end for forming substantially a clamp with which the surgeon may grip the needle and retract it towards him/herself once the suture has been completed. The plates have a tapered form so as to favour the introduction of the instrument towards the surgical site. Their facing surfaces may be provided with knurling or the like in order to firmly grip the needle.

Many different surgical instruments are known.

For example, DE 527 109 C describes a clamp. However, the Applicant has realized that the clamp in question is unable to grip and retain firmly a straight needle and does not have knurling on its gripping plate, useful for this purpose. The plate cannot be used for delicate blunt separation of the tissues (in particular indispensable for dissection of soft facial tissues). Moreover, there are no lateral slides which are able to convey remotely a suture mounted on a straight needle.

US 2013/090681 A1 describes a dilator device. However, the Applicant has realized that the device in question does not have a wide and blunt tip, but a pointed end suitable for perforating the tissues of the thoracic wall. The clamp therefore is unable to splay delicately facial tissues in a safe manner without causing injury. The clamp in question has a channel for conveying catheters, liquids, gases or the like, but does not have a lateral slide which allows positioning of the sutures provided on a straight needle. The instrument does not have a terminal surface able to grip and firmly hold straight needle sutures. WO 01/21231 A2 discloses another similar known prior art device.

EP 0,792,621 A1 describes a clamp with curved ends (such as a Backhaus clamp). The Applicant has realized that this clamp is totally inadequate for dissecting soft facial tissues, among other things because of its form, which does not have any type of plate or part making it suitable for performing a delicate dissection. The sutures which can be positioned with such an instrument can be modified only by gripping a tissue, but it is not possible to manipulate the suture needle after it has been extracted from the lateral slide. There is also no knurling at the ends useful for manoeuvring a straight needle once it has been extracted from the slide. DE 199 24 974 A1 discloses another similar known prior art device.

According to a first aspect of the invention a surgical instrument, comprising a first arm and a second arm pivoted to form substantially a first-class lever is provided, wherein:
the surgical instrument is configured to assume an open configuration, a closed configuration and a retaining clamped configuration for retaining a straight needle;
the first arm comprises a first handle grip at a first proximal end thereof and a first plate at a distal end thereof, the first plate having an outer dissecting surface and an inner retaining surface;
the second arm comprising a second handle grip at a proximal end thereof and a second plate at a distal end thereof, the second plate having an outer dissecting surface and an inner retaining surface;
at least one of the inner retaining surfaces being knurled so as to retain said needle in the retaining configuration;
the plates have a form tapered towards the distal end and a rounded outer surface;
said instrument also comprises a guide channel for guiding the straight surgical needle towards the distal end of at least one of said first arm and said second arm.

The tapered form of the plates allows, in the closed configuration of the instrument, the surgeon to penetrate towards the surgical site.

The rounded form of the plates facilitates use of the instrument in its dissecting configuration.

The knurled zones allows a needle to be gripped and retained and therefore suture operations to be performed.

The surgical instrument may have a channel on the outside of the arms. Preferably, however, the guide channel is internal, namely is formed at least partially in the thickness of at least one of the first arm and the second arm. The choice also depends on the material and the method with which the instrument is made. For example, the internal channel is advantageous when the instrument is of the disposable type and is for example made of plastic produced by means of moulding.

The guide channel may advantageously comprise at least one straight section.

In embodiments, the straight section of the guide channel comprises a longitudinal slot.

In embodiments, the guide channel comprises a curved section in the proximity of the retaining plate.

In embodiments, the guide channel comprises a distal opening on an outer face of the second plate of the second arm.

A spindle may also be provided for pushing the needle inside the guide channel.

In a preferred embodiment, the retaining plates are substantially drop-shaped or finger-nail shaped.

Each handle grip may be provided in the form of a ring.

A pushing surface may also be provided for pushing the instrument towards the operation site.

The surgical instrument may have the first arm which comprises a proximal portion shaped to form an open elbow.

The instrument, as mentioned above, may be of the disposable type. Alternatively, the instrument may be made of a metallic material.

The present invention, of which the scope is defined by the appended claims, will become clearer from the following description, provided by way of a non-limiting example, to be read with reference to the accompanying drawings, in which:
Figure 1 shows an instrument according to an embodiment of the present invention in an open configuration;
Figure 2 shows the instrument according to Figure 1 in the closed configuration;
Figure 3 shows in schematic form a needle with a thread inserted in its eye;
Figure 4 shows in schematic form a spindle for pushing the needle;

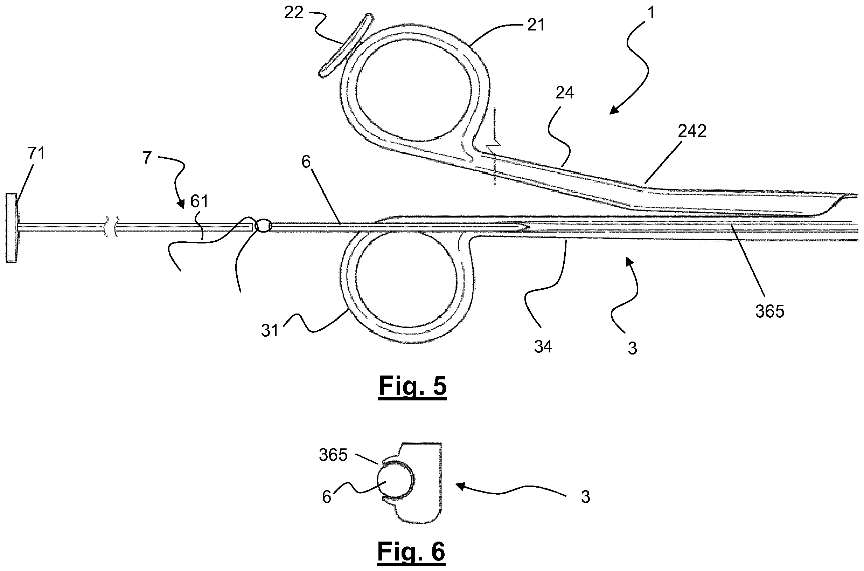

Figure 5 shows in schematic form the instrument with the needle inside the channel, pushed by the spindle;
Figure 6 shows a cross-section of the lower arm with spindle inserted inside the channel;
Figure 7a shows an outer face of the plate of the lower arm; and
Figure 7b shows the knurled inner face of the plate according to Figure 7a.

The instrument 1 comprises two arms 2, 3 pivoted to form substantially a first-class lever. The instrument 1 in particular comprises an upper arm 2, a lower arm 3 and a pivot point 4 for said arms.

Preferably, the upper arm 2 comprises a handle grip in the form of a ring 21. Similarly and preferably, the lower arm 3 comprises a handle grip in the form of a ring 31. Preferably, the ring 21 of the upper arm 2 is configured to be engaged by the thumb of a user's hand and the lower ring 31 is configured to be engaged by a middle finger of the user's hand.

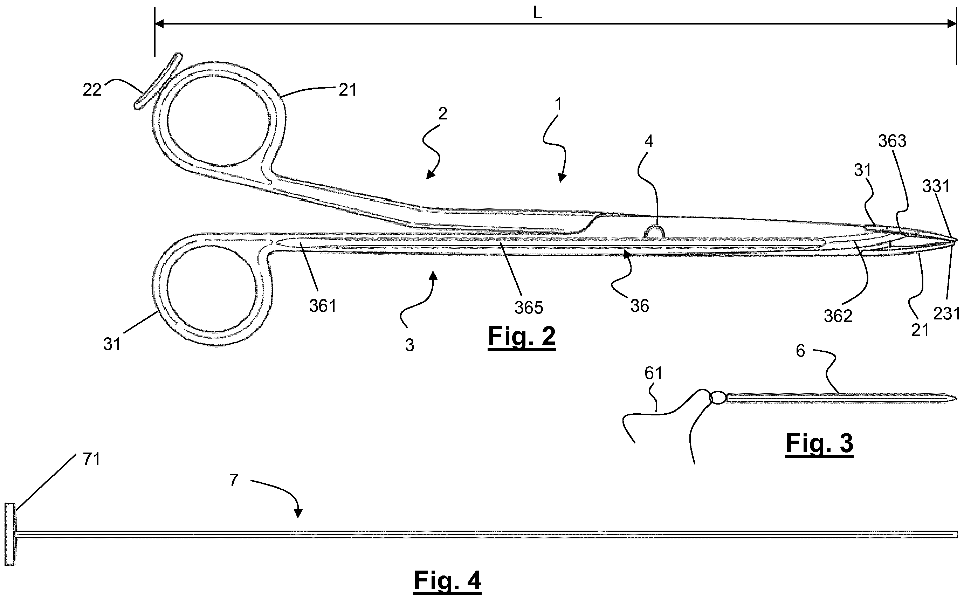

The instrument 1 may have a length L (Figure 2) ranging between about 130 mm and about 170 mm. In one embodiment the length L is equal to about 150 mm.

Preferably, the upper handle grip 21 comprises a pushing surface 22 for pushing the instrument 1 towards the operation site. The pushing surface 22 may comprise a rough concave surface, which is for example knurled or scored, said surface being shaped so as to seat comfortably the curved thumb of the user.

In the continuation of the present description, the adjective "proximal" will be used to indicate an element close to the handle grip 21, 31. On the contrary, the adjective "distal" will be used to indicate an element far from the handle grip 21, 31.

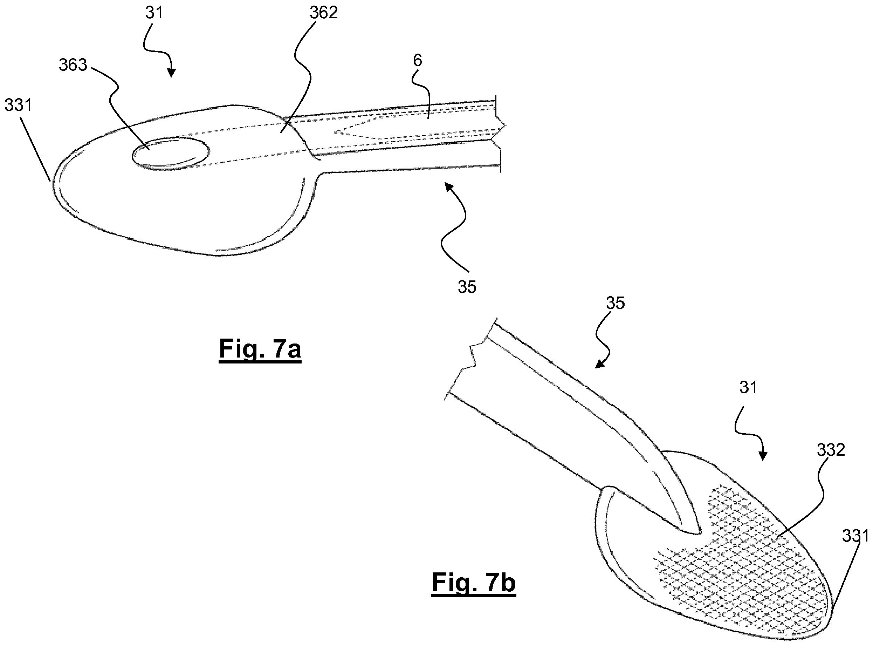

Preferably, each of the two arms 2,3 terminates in a plate 23, 33. According to embodiments, each plate 23, 33 has a drop-shaped form with the tip 231, 331 directed distally in relation to the handle grip 21, 31. When the instrument 1 is closed (Figure 2), the two plates 23, 33 are facing and in contact, at least at the tip 231, 331. Preferably, the inner surface of the plates is rough, at least in the proximity of their tip 231, 331. For example, the surface is knurled (as shown in Figure 7b, indicated by the reference number 332), scored or embossed. Preferably, the inner surface of the plates 23, 33 is substantially flat.

Each of the plates 23, 33 may have a length of about 10-15 mm and a width of about 5-10 mm. In one embodiment, each of the plates 23, 33 has a length of about 14 mm and a width of 8.5 mm. The tip of the plates is preferably knurled over a distance of about 3 mm.

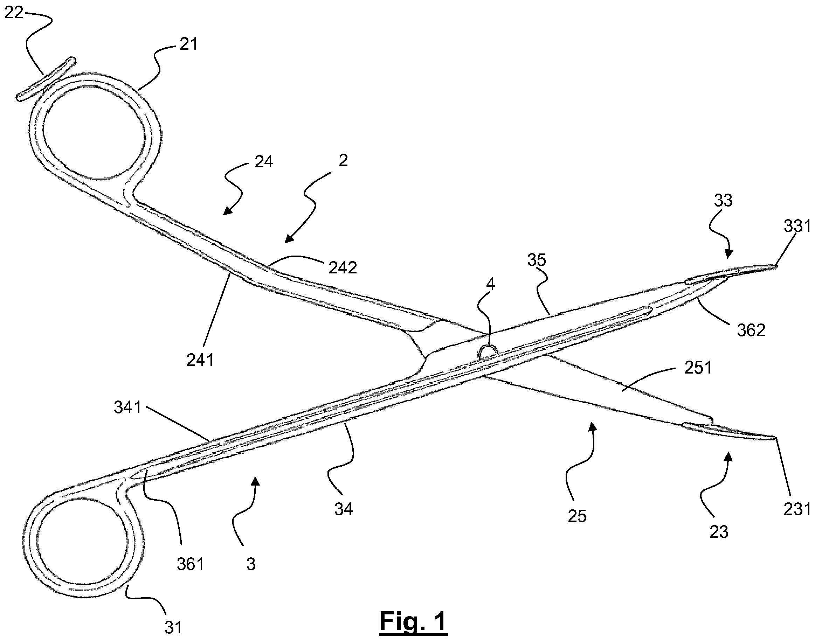

The lower arm 3 has preferably a substantially rectilinear axis and comprises a first proximal portion 34 and a second distal portion 35. The proximal portion 34 of the lower arm 3 comprises preferably an upper surface 341 which is substantially flat. The distal portion 35 of the lower arm 3 comprises preferably a substantially flat side surface (not shown in Figure 1 since opposite to the side surface visible in the same Figure 1).

The upper arm 2 comprises a first proximal portion 24 and a second distal portion 25. The proximal portion 24 of the upper arm 2 comprises preferably a lower surface 241 which is substantially flat. The distal portion 25 of the upper arm 2 comprises preferably a side surface 251 which is substantially flat. Preferably, the proximal portion 24 of the upper arm 2 is shaped so as to form an open elbow 242 with an angle of about 160° - 185°. In this way, even when the instrument is in the closed configuration (Figure 2), the handle grips 21, 31 are spaced and do not make contact with each other. This allows a greater closing force to be applied so as to grip firmly a needle between the two plates during a recovery manoeuvre (when the instrument is pulled by the surgeon towards him/herself), as will be explained more fully below.

The lower arm 3 and the upper arm 2 are pivoted. Preferably, they are pivoted at a point 4 along their distal portions. When pivotably assembled, the lower surface 241 of the upper arm 2 faces the lower surface 341 of the lower arm 3 and the side surfaces 251, 351 of the distal portions 25, 35 face each other in a similar manner to the two blades of scissors.

When the arms 2, 3 of the instrument 1 are closed (Figure 2), the instrument has a tapered form and a cross-section with rounded-off angles.

Preferably, the lower arm 3 comprises a guide channel 36 substantially along its entire length. Preferably, the guide channel 36 has a cross-section which is substantially constant, for example substantially circular, and a substantially rectilinear axis along substantially its entire length, except for the distal portion 362. Along the distal portion 362 the guide channel curves gently towards the plate 33.

Preferably, the proximal opening 361 of the guide channel 36 is in the proximity of the handle grip 31 of the lower arm 3 and is cut diagonally so as to create an insertion guide surface. The guide channel 36 is open (363) along the drop-shaped plate 33 at the end of the distal portion 35 of the lower arm 3. Preferably, the distal opening 363 of the guide channel 36 is located on the upper face of the drop-shaped plate 33, as shown in Figure 7. The guide channel 36 is preferably arranged on the right-hand side, when the device 1 is gripped by the right hand of a user.

The channel may have a closed cross-section along its entire length or may be open substantially along the whole (or part of) its length, except for the distal portion 362. The opening in the guide channel which extends longitudinally is indicated in the figures by the number 365.

The guide channel 36 is configured to guide a surgical needle 6 (Figure 3) from its proximal opening 361 to its distal opening 363, when the instrument 1 is positioned in the surgical site.

Preferably, in order to push a surgical needle from the proximal opening 361 to the distal opening 363 of the guide channel 36, a spindle 7 with a grip 71 for better gripping thereof and for pushing the needle 6 is provided.

In order to perform a surgical operation, for example a cosmetic surgical operation, the instrument 1 is pushed into the surgical site, for example passing through a suitable incision. The surgeon has the possibility of reaching the exact position while keeping the instrument closed (and therefore taking advantage of its slim and tapered form) or raising other organs, muscles or tendons, by slightly opening the handle grips of the instrument.

Once the precise position has been reached, the surgical needle 6, with a surgical thread 61 inserted in its eye, is introduced into the guide channel 36 and, preferably, is pushed towards the distal opening 363 of the channel 36 by means of the spindle 7.

Once the needle 6 has been extracted from the distal opening 363, the needle may penetrate through an organ and/or a tissue to be stitched together with a suture. The surgeon may grip the needle passing out from the tissue and perform a suture by introducing the needle in the opposite direction. When the needle is introduced again into the tissue close to the point from where it emerged, it may be clamped firmly between the two drop-shaped plates 21, 23. The surgeon, keeping the arms of the instrument firmly closed, may pull towards him/herself the instrument 1 with the needle. In this way a suture is performed in the desired position.

Advantageously, the distal portions 25, 35 of the arms 2 and 3 have a length smaller than that of the proximal arms 24, 34. This allows one to apply greater force and overcome any obstacles encountered in order to reach the operation site.

## Claims

1. A surgical instrument (1), comprising a first arm (2) and a second arm (3) pivoted to form substantially a first-class lever, wherein
said surgical instrument is configured to assume an open configuration, a closed configuration and a retaining clamped configuration for retaining a straight needle;
the first arm (2) comprising a first handle grip (21) at a proximal end thereof and a first plate (23) at a distal end thereof, the first plate (23) having an outer dissecting surface and an inner retaining surface;
the second arm (3) comprising a second handle grip (31) at a proximal end thereof and a second plate (33) at a distal end thereof, the second plate (33) having an outer dissecting surface and an inner retaining surface;
at least one of the inner retaining surfaces being knurled (332) so as to retain said needle in the retaining configuration;
the plates (23, 33) have a form tapered towards the distal end and a rounded outer surface;
said instrument (1) also comprises a guide channel (36) for guiding the straight surgical needle (6) towards the distal end of at least one of said first arm (2) and said second arm (3).

2. The surgical instrument (1) according to claim 1, wherein said guide channel (36) is formed at least partially in the thickness of at least one of said first arm (2) and said second arm (3).

3. The surgical instrument (1) according to claim 1 or 2, wherein said guide channel (36) comprises at least one straight section.

4. The surgical instrument (1) according to claim 3, wherein said straight section of the guide channel (36) comprises a longitudinal slot (365).

5. The surgical instrument (1) according to any one of the preceding claims, wherein said guide channel (32) comprises a curved section (362) in the proximity of said retaining plate (33).

6. The surgical instrument (1) according to any one of the preceding claims, wherein said guide channel (36) comprises a distal opening (363) on an outer face of the second plate (33) of the second arm (3).

7. The surgical instrument (1) according to any one of the preceding claims, further comprising a spindle (7) for pushing the needle (6) inside the guide channel (36).

8. The surgical instrument (1) according to any one of the preceding claims, wherein said retaining plates are substantially drop-shaped.

9. The surgical instrument (1) according to any of the preceding claims, wherein each of said handle grips (21, 31) comprises a ring.

10. The surgical instrument (1) according to any one of the preceding claims, further comprising a pushing surface (22) for pushing the instrument (1) towards the operation site.

11. The surgical instrument (1) according to any one of the preceding claims, wherein said first arm (2) comprises a proximal portion (24) shaped so as to form an open elbow (242).

12. The surgical instrument (1) according to any one of the preceding claims, wherein said instrument is of the disposable type.

13. The surgical instrument (1) according to any one of claims 1-11, wherein said instrument is made of a metallic material.

## Patentansprüche

1. Chirurgisches Instrument (1), umfassend einen ersten Arm (2) und einen zweiten Arm (3), die schwenkbar sind, um im Wesentlichen einen Hebel der ersten Klasse zu bilden, wobei
das chirurgische Instrument konfiguriert ist, um eine offene Konfiguration, eine geschlossene Konfiguration und eine eingespannt gehaltene Konfiguration anzunehmen zum Halten einer geraden Nadel;
der erste Arm (2) einen ersten Handgriff (21) an einem proximalen Ende davon und eine erste Platte (23) an einem distalen Ende davon umfasst, wobei die erste Platte (23) eine äußere Präparierfläche und eine innere Haltefläche aufweist; wobei der zweite Arm (3) einen zweiten Handgriff (31) an einem proximalen Ende davon und eine zweite Platte (33) an einem distalen Ende davon umfasst, wobei die zweite Platte (33) eine äußere Präparierfläche und eine innere Haltefläche aufweist;
mindestens eine der inneren Halteflächen gerändelt (332) ist, um diese Nadel in der Haltekonfiguration zu halten;
die Platten (23, 33) eine sich zum distalen Ende hin verjüngende Form und eine abgerundete Außenfläche haben;
das Instrument (1) auch einen Führungskanal (36) zum Führen der geraden, chirurgischen Nadel (6) zum distalen Ende von mindestens einem des ersten Arms (2) und des zweiten Arms (3) umfasst.

2. Chirurgisches Instrument (1) nach Anspruch 1, wobei dieser Führungskanal (36) zumindest teilweise in der Dicke von zumindest einem, entweder des ersten Arms (2) oder des zweiten Arms (3) ausgebildet ist.

3. Chirurgisches Instrument (1) nach Anspruch 1 oder 2, wobei der Führungskanal (36) mindestens einen geraden Abschnitt umfasst.

4. Chirurgisches Instrument (1) nach Anspruch 3, wobei der gerade Abschnitt des Führungskanals (36) einen Längsschlitz (365) umfasst.

5. Chirurgisches Instrument (1) nach einem der vorhergehenden Ansprüche, wobei der Führungskanal (32) einen gekrümmten Abschnitt (362) in der Nähe dieser Halteplatte (33) umfasst.

6. Chirurgisches Instrument (1) nach einem der vorhergehenden Ansprüche, wobei der Führungskanal (36) eine distale Öffnung (363) an einer Außenfläche der zweiten Platte (33) des zweiten Arms (3) umfasst.

7. Chirurgisches Instrument (1) nach einem der vorhergehenden Ansprüche, ferner umfasst eine Spindel (7) zum Schieben der Nadel (6) in den Führungskanal (36) hinein.

8. Chirurgisches Instrument (1) nach einem der vorhergehenden Ansprüche, wobei die Halteplatten im Wesentlichen tropfenförmig sind.

9. Chirurgisches Instrument (1) nach einem der vorhergehenden Ansprüche, wobei jeder der Handgriffe (21, 31) einen Ring umfasst.

10. Chirurgisches Instrument (1) nach einem der vorhergehenden Ansprüche, ferner umfasst eine Schiebefläche (22) zum Schieben des Instruments (1) in Richtung der Operationsstelle.

11. Chirurgisches Instrument (1) nach einem der vorhergehenden Ansprüche, wobei der erste Arm (2) einen proximalen Abschnitt (24) umfasst, der so geformt ist, dass er ein offenes Kniestück (242) bildet.

12. Chirurgisches Instrument (1) nach einem der vorhergehenden Ansprüche, wobei das Instrument vom Einwegtyp ist.

13. Chirurgisches Instrument (1) nach einem der Ansprüche 1 bis 11, wobei das Instrument aus einem metallischen Material hergestellt ist.

## Revendications

1. Instrument chirurgical (1), comprenant un premier bras (2) et un second bras (3) pivotant pour former sensiblement un levier de première catégorie, dans lequel
ledit instrument chirurgical est configuré pour prendre une configuration ouverte, une configuration fermée et une configuration serrée de retenue pour retenir une aiguille droite ;
le premier bras (2) comprenant une première poignée (21) à une extrémité proximale de celui-ci et une première plaque (23) à une extrémité distale de celui-ci, la première plaque (23) ayant une surface de dissection externe et une surface de retenue interne ;
le second bras (3) comprenant une seconde poignée (31) à une extrémité proximale de celui-ci et une seconde plaque (33) à une extrémité distale de celui-ci, la seconde plaque (33) ayant une surface de dissection externe et une surface de retenue interne ;
au moins l'une des surfaces de retenue internes étant moletée (332) de sorte à retenir ladite aiguille dans la configuration de retenue ;
les plaques (23, 33) ont une forme conique vers l'extrémité distale et une surface externe arrondie ;
ledit instrument (1) comprend également un canal de guidage (36) pour guider l'aiguille chirurgicale droite (6) vers l'extrémité distale d'au moins l'un dudit premier bras (2) et dudit second bras (3).

2. Instrument chirurgical (1) selon la revendication 1, dans lequel ledit canal de guidage (36) est formé au moins partiellement dans l'épaisseur d'au moins l'un dudit premier bras (2) et dudit second bras (3).

3. Instrument chirurgical (1) selon la revendication 1 ou 2, dans lequel ledit canal de guidage (36) comprend au moins une section droite.

4. Instrument chirurgical (1) selon la revendication 3, dans lequel ladite section droite du canal de guidage (36) comprend une fente longitudinale (365).

5. Instrument chirurgical (1) selon l'une quelconque des revendications précédentes, dans lequel ledit canal de guidage (32) comprend une section incurvée (362) à proximité de ladite plaque de retenue (33).

6. Instrument chirurgical (1) selon l'une quelconque des revendications précédentes, dans lequel ledit canal de guidage (36) comprend une ouverture distale (363) sur une face externe de la seconde plaque (33) du second bras (3).

7. Instrument chirurgical (1) selon l'une quelconque des revendications précédentes, comprenant en outre une tige (7) pour pousser l'aiguille (6) à l'intérieur du canal de guidage (36).

8. Instrument chirurgical (1) selon l'une quelconque des revendications précédentes, dans lequel lesdites plaques de retenue sont sensiblement en forme de gouttes.

9. Instrument chirurgical (1) selon l'une quelconque des revendications précédentes, dans lequel chacune desdites poignées (21, 31) comprend un anneau.

10. Instrument chirurgical (1) selon l'une quelconque des revendications précédentes, comprenant en outre une surface de poussée (22) pour pousser l'instrument (1) vers le site d'opération.

11. Instrument chirurgical (1) selon l'une quelconque des revendications précédentes, dans lequel ledit premier bras (2) comprend une partie proximale (24) profilée de sorte à former un coude ouvert (242).

12. Instrument chirurgical (1) selon l'une quelconque des revendications précédentes, dans lequel ledit instrument est du type jetable.

13. Instrument chirurgical (1) selon l'une quelconque des revendications 1 à 11, dans lequel ledit instrument est composé d'un matériau métallique.
